**Europäisches Patentamt**

**European Patent Office**

**Office européen des brevets**

(11) Veröffentlichungsnummer: **0 329 017**

**A1**

# (12) EUROPÄISCHE PATENTANMELDUNG

(21) Anmeldenummer: **89102241.0**

(22) Anmeldetag: **09.02.89**

(51) Int. Cl.⁴: **C03C 10/16 , A61K 6/06 , C03C 4/00**

(30) Priorität: **15.02.88 DD 312888**

(43) Veröffentlichungstag der Anmeldung:
**23.08.89 Patentblatt 89/34**

(84) Benannte Vertragsstaaten:
**CH DE FR GB IT LI SE**

(71) Anmelder: **VEB
WISSENSCHAFTLICH-TECHNISCHES WERK
BAD MUSKAU
Heideweg 2
DDR-7582 Bad Muskau(DD)**

(72) Erfinder: **Abert, Christine, Dr. med.
Habersaathstrasse 24 B
DDR-1020 Berlin(DD)**
Erfinder: **Breustedt, Alfred, Prof. Dr.sc.med.
Mollstrasse 15
DDR-1020 Berlin(DD)**

Erfinder: **Grosse, Steffen, Dipl.-Med.
Egon-Erwin-Kisch-Strasse 77
DDR-1090 Berlin(DD)**
Erfinder: **Müller, Henry, Dipl.-Med.
Linienstrasse 115
DDR-1040 Berlin(DD)**
Erfinder: **Vogel, Frank, Dipl.-Med.
Hermann-Tops-Strasse 37
DDR-1800 Brandenburg(DD)**
Erfinder: **Lätsch, Wolfgang
Gersdorfer Strasse 87
DDR-8907 Reichenbach(DD)**

(74) Vertreter: **Patentanwälte Beetz sen. - Beetz
jun. Timpe - Siegfried - Schmitt-Fumian-
Mayr
Steinsdorfstrasse 10
D-8000 München 22(DE)**

(54) **Glaskeramisches Material mit Cordierit-Kristallphase und seine Verwendung.**

(57) Das glaskeramische Material mit Cordierit-Kristallphase ist für medizinische, vorzugsweise stomatologische Zwecke geeignet. Mit diesem Material wird es ermöglicht, bei einer ausreichenden Belastbarkeit und biologischen Verträglichkeit eine dem Naturprodukt weitgehend angenäherte äußere Form und Farbgebung auszubilden.

Überraschenderweise wurde gefunden, daß dieses durch ein Material folgender Zusammensetzung

| | | | |
|---|---|---|---|
| $SiO_2$ | = | 40 - 48 | Gew.% |
| $Al_2O_3$ | = | 23 - 26 | " |
| MgO | = | 8 - 12 | " |
| $Na_2O$ | = | 3,0 - 5,5 | " |
| $K_2O$ | = | 0 - 6,0 | " |
| CaO | = | 0,01 - 0,2 | " |
| $F^-$ | = | 2,9 - 4,0 | " |
| $Cl^-$ | = | 0,01 - 0,5 | " |
| $P_2O_5$ | = | 2,0 - 6,0 | " |
| CoO | = | 0,0001 - 0,001 | " |

erreicht wird.

EP 0 329 017 A1

Die Verwendung des glaskeramischen Materials erfolgt als stomatologisches Produkt, wie Inlays, Kronen, Brücken, Aufbauten, Schalen und medizinische Unikate.

## Glaskeramisches Material mit Cordierit-Kristallphase und seine Verwendung

Die Erfindung bezieht sich auf ein glaskeramisches Material mit Cordierit-Kristallphase für medizinische, vorzugsweise stomatologische Zwecke, und seine Verwendung.

Es ist bekannt, zerstörte Zähne in der Stomatologie mit Hilfe spezifischer Prothesen zu restaurieren. Diese Prothesen, vorzugsweise Inlays, Kronen, Brücken und Zahnaufbauten,werden aus körperfremdem Material hergestellt. Ein ideales Material, das den Ansprüchen der Ästhetik, der Belastbarkeit, der biologischen Verträglichkeit und der Forderung nach einer ökonomisch günstigen Technologie in allen Punkten genügt, ist derzeit noch nicht vorhanden. Die am häufigsten verwendeten Werkstoffe sind gegenwärtig noch Edelmetallegierungen und edelmetallfreie Legierungen. Ihr Vorteil liegt in der leichten Verarbeitung mittels Schleudergußtechnologie und der guten Paßfähigkeit. Die ungenügende Ästhetik, ihr hoher Preis und die fragwürdige biologische Verträglichkeit der Nichtedelmetallegierungen lassen den Wunsch nach Alternativmaterialien entstehen. Organische Polymere und deren Kompositionswerkstoffe haben eine relativ geringe Festigkeit. Vor allem ihr niedriger Elastizitätsmodul und der höhere thermische Ausdehnungskoeffizient, die sie gegenüber dem Zahnschmelz besitzen, führen zu einer kurzen Anwendungszeit der Restaurationen aus Polymeren. Die geringe Abriebfestigkeit macht sie im Seitenzahngebiet nur begrenzt einsetzbar. Für hohe ästhetische Ansprüche im Frontzahngebiet werden Sinterkeramiken auf der Basis von Feldspatgläsern eingesetzt. Die geringen Biegefestigkeitswerte von maximal 70 MPa bedingen ihre enge Indikationsstellung für Zähne, die nur relativ geringen Belastungen ausgesetzt sind. Während die keramischen Materialien und Herstellungsverfahren zwar traditionell sind, aber die Präzision der Passung (auf Urmodell) schwer, bei Inlays überhaupt nicht, herzustellen ist, bieten Materialien einen Vorteil, die nach dem Schleudergußverfahren in verlorene Wachsformen verarbeitet werden können.

Aus diesem Grund stellen glaskeramische Materialien eine echte Alternative dar. Ein derartiges Material ist in der DD-PS 242 172 in Form einer Glimmer-Cordierit-Glaskeramik beschrieben. Diese weist jedoch ein unnatürliches, weißlichopakes Aussehen auf. Weiterhin tritt eine bräunlich-gräulich marmorartige Veränderung der Glaskeramik bei Glasur und kurzzeitiger Temperaturbehandlung über 600 °C auf. Aufgrund dieser Verfärbungen und ihres opaken Aussehens ist diese Glaskeramik unbrauchbar für eine individuelle Farbgestaltung ähnlich der natürlicher Zähne. Diese Nachteile machen die erwähnte Glaskeramik als Restaurationsmaterial in der Stomatologie aus ästhetischen Gründen wenig geeignet. Diese bekannte Glimmer-Cordierit-Glaskeramik besitzt außerdem Röntgenopazität. Diese ist eine Eigenschaft, die in der modernen Zahnmedizin nicht angestrebt wird, da so unter den Restaurationen befindliche kariöse Defekte durch eine Röntgenaufnahme nicht sichtbar gemacht werden können.

In der DD-PS 242 172 wird die Eigenschaft des Materials hervorgehoben, daß seine Abrasion der der natürlichen Zähne ähnlicher wäre als die von in der Stomatologie verwendeten Legierungen. Die Glimmer-Cordierit-Glaskeramik zeigt jedoch im Abriebtest eine wesentlich höhere Abrasion als bekannte Legierungen ("Sipal", "NCA-Gisadent") sowie Dentalkeramiken.

Es ist bekannt, daß die Eigenschaften von Glaskeramiken im wesentlichen durch deren Hauptkristallphasen und den Anteil der Restglasphase bestimmt werden. Als Hauptkristallphasen werden für restaurative Dentalmaterialien gegenwärtig angegeben:
- Leucit, Lithiumalumosilicat, Glimmer, Glimmer/Cordierit, Calciumphosphatkristallphasen, Apatit, Apatit/Wollastonit.

In der DE-PS 3 435 348 ist eine Glaskeramik mit apatitischer Kristallphase beschrieben. Aus der Beschreibung geht hervor, daß eine Wollastonitbildung vermieden werden sollte, da Wollastonit des glaskeramische Produkt beschädigen kann.

Auch in der JP-PS 57-191252 wird auf die nachteilige Wirkung von Wollastonit in gegossenen Glaskeramikprodukten hingewiesen. Nachteilig bei beiden Lösungen sind die geringen Biegebruchfestigkeitswerte und eine nachträglich schwer vorzunehmende Farbkorrektur des Zahnersatzmaterials.

Die in der US-PS 4 431 420 und der EP-PS 0 022 655 beschriebene Glaskeramik besitzt ebenfalls Nachteile, wie sich im klinischen Einsatz herausstellte. So wird die natürliche Fluoreszenz des Zahnschmelzes durch die Glaskeramik auf Tetrakieselsäureglimmerbasis nicht realisiert.

Der Erfindung liegt die Aufgabe zugrunde, ein glaskeramisches Material mit Cordierit-Kristallphase für medizinische, vorzugsweise stomatologische Zwecke, und seine Verwendung zu schaffen, welches es ermöglicht, bei einer ausreichenden Belastbarkeit und biologischen Verträglichkeit eine dem Naturprodukt weitgehend angenäherte Form und Farbgebung auszubilden.

Überraschenderweise wurde gefunden, daß die vorgenannte Aufgabenstellung durch ein glaskeramisches Material mit Cordierit-Kristallphase folgender Zusammensetzung

3

| SiO$_2$ | = | 40 - 48 | Gew.% |
|---------|---|---------|-------|
| Al$_2$O$_3$ | = | 23 - 26 | " |
| MgO | = | 8 - 12 | " |
| Na$_2$O | = | 3,0 - 5,5 | " |
| K$_2$O | = | 0 - 6,0 | " |
| CaO | = | 0,01- 0,2 | " |
| F$^-$ | = | 2,9 - 4,0 | " |
| Cl$^-$ | = | 0,01- 0,5 | " |
| P$_2$O$_5$ | = | 2,0 - 6,0 | " |
| CoO | = | 0,0001- 0,001 | " |

gelöst wird.

Gegebenenfalls kann als Nebenkristallphase Spinell auftreten. Durch die Zugabe von CoO in geringen Prozentzahlen konnte überraschend ein natürlicher Transparenzeffekt erreicht werden. Durch diesen Effekt und das optimale Keramisierprogramm zur Transparenz, die bei dem erfindungsgemäßen glaskeramischen Material bei 50 - 70 % liegen kann, tritt überraschend der erwünschte Chamäleoneffekt auf. Hierbei kann das glaskeramische Material Zusatzkomponenten für die Farbgebung, insbesondere Metalloxide, enthalten.

In den Bereichen, in denen besonders hohe Anforderungen an die Ästhetik gestellt werden, kann das Material Zusatzkomponenten in Form von seltenen Erden zur Erzielung einer Fluoreszenz ähnlich der natürlicher Zähne enthalten. Das glaskeramische Material kann auch eine Zusatzkomponente aus ZrO$_2$ von 0,2 - 0,25 Gew.% zur Erzielung einer Fluoreszenz enthalten. Es ist auch möglich, daß es durch Malfarben in Form farbiger Glasuren und Beizen farbig gestaltet ist. Dabei ist es kompatibel mit herkömmlichen Malfarben der Ganzkeramiktechnologie.

In weiterer Ausgestaltung der Erfindung wird das überraschenderweise gefundene glaskeramische Material mit Cordierit-Kristallphase als stomatologisches Produkt, wie Inlays, Kronen, Brücken, Aufbauten, Schalen, medizinische Unikate, verwendet. Dabei kann das glaskeramische Material in reiner, kompakter Form oder als Ummantelung oder in Form des Grundkörpers benutzt werden. Es wurde gefunden, daß die Kombination mit hochfesten Werkstoffen, wie Metallen und Sinterkeramiken, möglich ist.

Durch die Erzeugung spezieller Eigenschaften, wie einstellbarerAusdehnungskoeffizienten, vorzugsweise eines Wärmeausdehnungskoeffizienten von 4 bis 9 . 10$^{-6}$/K, haben Restaurationen mit dem erfindungsgemäßen Material die gleiche gute paßfähigkeit wie Metallgußobjekte.

Die erreichbare mechanische Belastbarkeit von stomatolo gischen Produkten bis zu Biegebruchfestigkeitswerten von 320 MPa, machen sie zu einem idealen Material für das Seitenzahngebiet. Im Frontzahngebiet und überall dort, wo hohe ästhetische Ansprüche gestellt werden, sind die glaskeramischen Restaurationen durch die vom Material erzeugte Fluoreszenz durch Beigabe von seltenen Erden besonders geeignet.

Das erfindungsgemäße Material ist bioinert und für das Mundhöhlenmilieu biokompatibel. Außerdem läßt es sich nach dem in der Zahntechnik am meisten verwendeten Schleudergußverfahren in an sich bekannter Weise verarbeiten. Weitere überraschend nachgewiesene Eigenschaften des Materials sind beispielsweise die Einfärbbarkeit bis hin zum Prozeß der Keramisierung und der Kombination mit hochfesten Werkstoffen, wie Metallen und Sinterkeramiken. Das erfindungsgemäße glaskeramische Material mit Cordierit-Kristallphase läßt sich mit Korundsteinchen, Gummipolierern und ähnlichen in der Stomatologie üblichen Werkzeugen an seiner Oberfläche bearbeiten und polieren.

In einem Ausführungsbeispiel wird als eine mögliche Verwendung der Erfindung eine Kronenrestauration mit dem erfindungsgemäßen material näher erläutert:

Der Zahnarzt präpariert einen Zahnstumpf in der Art, daß eine Schichtstärke des Restaurationsmittels von mindestens 1 mm resultieren kann. Dabei sind im besonderen die approximalen, vestibulären und oralen Flächen des natürlichen Zahnes mindestens 1 mm und die Okklusalfläche mindestens 1,5 mm zu reduzieren. Die Präparationsgrenze wird in Form einer Stufe oder einer Hohlkehle geschaffen. Nach erfolgter Abformung mit an sich bekannten Abformmaterialien und -methoden wird der Abdruck mit einem Stumpfmaterial, insbesondere einem Spezialhartgips Typ IV, ausgegossen und somit ein Modellstumpf des natürlichen Zahnes geschaffen, der die Verhältnisse im Munde des Patienten eindeutig wiedergibt. Auf diesem erfolgt die Modellation des späteren Restaurationsmittels aus einem Modellierwachs beziehungsweise Kunststoff.

Als vorteilhaft erweist sich hierbei die Verwendung einer Platzhalterfolie. Der Gußkanal mit einem Durchmesser größer als oder gleich 3,5 mm und einer Länge kleiner als oder gleich 4 mm, bestehend aus dem gleichen Material wie die Modellation, wird am modellierten Gußobjekt angebracht. Luftabzugskanäle

4

können zur Vermeidung von Lufteinschlüssen beim Guß gestaltet werden. Zur Herstellung der Gußhohlform wird die Wachsmodellation mit einer an sich bekannten aushärtbaren Einbettmasse umgeben. Diese Einbettmasse besteht vorzugsweise aus Quarz oder einer seiner Modifikationen und Magnesiumphosphaten als Bindemittel. Diese Gußhohlform wird auf 600 - 900 °C erhitzt und ist dann für den formfüllenden Guß mit der Grundglasschmelze des erfindungsgemäßen Materials geeignet.

Die Schmelz- bzw. Keramisiertechnologie wird für alle zu gießenden Objekte nachfolgend beschrieben, da sie ihren Bedingungen nach für jede Anwendung gleich ist.

Die Grundglasfritte wird in Tiegeln, deren Material sich gegenüber der Silikatschmelze inert verhalten muß, bei 1550 °C erschmolzen. Dann erfolgt das formfüllende Einbringen der Glasschmelze in die Hohlform, beispielsweise mittels Schleuderkraft oder auch durch Über- und/oder Unterdruck. Nach dem Abkühlen bis auf Raumtemperatur unter genormten Bedingungen wird das entstandene Glasobjekt von der umhüllenden Einbettmasse befreit. Nachfolgend dann das glasklare Objekt auf seine Paßfähigkeit auf dem Modell und auf eventuelle Gußfehler, z. B. Schlieren, Blasen, Fremdkörpereinschlüsse, visuell geprüft werden.

Anschließend wird das Gußobjekt zum Überführen in den semikristallinen Zustand einer gesteuerten Temperaturführung ausgesetzt. Das Gußobjekt wird zur Wahrung seiner Formstabilität von einer geeigneten Keramisiereinbettmasse umhüllt. Diese muß ein thermisches Ausdehnungsverhalten analog dem des Ausgangsglases bis $T_G$ zeigen. Durch Erwärmen mit 10 K/min auf eine Temperatur von 700 - 1100 °C über eine Zeit von 1 - 24 h erfolgt die Keramisierung in das Produkt aus dem erfindungsgemäßen Material. Eine Transparenz des Produktes in der erfindungsgemäßen Zusammensetzung von 65 - 75% ist beispielsweise bei folgender Temperaturführung zu erzielen:
- Keramisieren 1 Stunde bei 710 °C und 4 Stunden bei 960 °C. Die Aufheizgeschwindigkeit beträgt 20 K/min. Nach Abkühlen auf Raumtemperatur mit 1 K/min bei definierten Abkühlbedingungen wird die glaskeramische Restauration von der Keramisiereinbettmasse befreit. Ein Ausarbeiten mit herkömmlichen Schleifkörpern oder Polieren beziehungsweise Bemalen ist möglich. Eine individuelle Farbgebung kann mit Unter- und/oder Aufglasurfarben erzielt werden.

Tabelle 1

| Zusammensetzungen von glaskeramischen Materialien mit Cordierit-Kristallphase bzw. deren Ausgangsgläsern | | |
|---|---|---|
| | 1 | 2 |
| $SiO_2$ | 44 - 47 | 42 - 46 |
| $Al_2O_3$ | 24 - 25 | 25 - 26 |
| $MgO$ | 8 - 10 | 9 - 11 |
| $Na_2O$ | 4,0 - 5,5 | 3,5 - 5,0 |
| $K_2O$ | - | 0,2 - 0,4 |
| $CaO$ | 0,01 - 0,2 | 0,1 - 0,2 |
| $F^-$ | 3,0 - 3,5 | 3,5 - 4,0 |
| $Cl^-$ | 0,1 - 0,3 | 0,01 - 0,2 |
| $P_2O_5$ | 2,0 - 5,0 | 4,0 - 6,0 |
| $CoO$ | 0,0008 - 0,001 | 0,0001 - 0,0005 |
| $ZrO_2$ | - | 0,2 - 0,25 |

Tabelle 2

| Eigenschaften und Herstellung | | |
|---|---|---|
| Variante Nr. | 1 | 2 |
| Temperatur<br>Temperzeit | 700 °C; 900 °C<br>1 h; 4 h | 710 °C; 960 °C<br>1 h; 4 h |
| linearer therm. $\alpha$-Wert ($K^{-1}$)<br>Biegebruchf.[1] (MPa)<br>Farbe<br>Transparenz | 387,7<br>90<br>hellblau<br>75 % | 597,0<br>160<br>weißlich-blau<br>70 % |

[1] Probekörper wurden nach dem Schleudergußverfahren hergestellt.

Die in der Tabelle 1 und 2 näher definierten glaskeramischen Materialien weisen eine für stomatologische Zwecke besonders geeignete Zusammensetzung auf, ohne dabei jedoch die erfindungsgemäße Lösung dadurch einzuschränken. Der Zusatz von 0,2 - 0,25 Gew.% $ZrO_2$ gewährleistet eine Erhöhung der Fluoreszenz bei Zähnen, wie sie besonders auch im Frontzahngebiet erwünscht ist.

## Ansprüche

1. Glaskeramisches Material mit Cordierit-Kristallphase für medizinische, vorzugsweise stomatologische Zwecke, dadurch gekennzeichnet, daß es folgende Zusammensetzung aufweist:

| | | | |
|---|---|---|---|
| $SiO_2$ | = | 40 - 48 | Gew.% |
| $Al_2O_3$ | = | 23 - 26 | " |
| $MgO$ | = | 8 - 12 | " |
| $Na_2O$ | = | 3,0 - 5,5 | " |
| $K_2O$ | = | 0 - 6,0 | " |
| $CaO$ | = | 0,01 - 0,2 | " |
| $F^-$ | = | 2,9 - 4,0 | " |
| $Cl^-$ | = | 0,01 - 0,5 | " |
| $P_2O_5$ | = | 2,0 - 6,0 | " |
| $CoO$ | = | 0,0001 - 0,001 | " |

2. Glaskeramisches Material nach Anspruch 1, dadurch gekennzeichnet, daß es Zusatzkomponenten für die Farbgebung, insbesondere Metalloxide, enthält.

3. Glaskeramisches Material nach Anspruch 1, dadurch gekennzeichnet, daß es durch Malfarben in Form farbiger Glasuren und Beizen farbig gestaltet ist.

4. Glaskeramisches Material nach Anspruch 1, dadurch gekennzeichnet, daß es Zusatzkomponenten in Form von seltenen Erden zur Erzielung einer Fluoreszenz ähnlich der natürlichen Zähne enthält.

5. Glaskeramisches Material nach Anspruch 1, dadurch gekennzeichnet, daß es eine Zusatzkomponente aus $ZrO_2$ von 0,2 - 0,25 Gew.% zur Erzielung einer Fluoreszenz enthält.

6. Glaskeramisches Material nach Anspruch 1, dadurch ge kennzeichnet, daß das glaskeramische Material eine Transparenz von 50 bis 70 % aufweist.

7. Verwendung des glaskeramischen Materials mit Cordierit-Kristallphase nach einem oder mehreren der Ansprüch 1 bis 6 als stomatologisches Produkt, wie Inlays, Kronen, Brücken, Aufbauten, Schalen und medizinische Unikate.

8. Verwendung nach Anspruch 7, dadurch gekennzeichnet, daß das glaskeramische Material in reiner kompakter Form oder als Ummantelung oder in Form des Grundkörpers benutzt wird.

9. Verwendung nach Anspruch 7, dadurch gekennzeichnet, daß das Produkt einen Wärmeausdehnungskoeffizienten von 4 bis 9 . $10^{-6}$/K, eine Biegebruchfestigkeit von 50 bis 320 MPa, Röntgentransluzenz aufweist und biokompatibel ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 225 279 (VEB JENAER GLASWERK) <br> * Ansprüche 1-3 * <br> --- | 1-9 | C 03 C 10/16 <br> A 61 K 6/06 <br> C 03 C 4/00 |
| Y | EP-A-0 077 223 (BOUSSOIS S.A.) <br> * Anspruch 1 * <br> --- | 1-9 | |
| Y | DE-A-2 815 312 (SAALE-GLAS GmbH) <br> * Ansprüche 1-4; Seite 7, Zeilen 10-17 * <br> --- | 1-9 | |
| Y,D | DD-A- 242 172 <br> (FRIEDRICH-SCHILLER-UNIVERSITÄT JENA) <br> * Ansprüche 1,2 * <br> ----- | 1-9 | |

RECHERCHIERTE
SACHGEBIETE (Int. Cl.4)

C 03 C
A 61 K

Der vorliegende Recherchenbericht wurde für alle Patentansprüche erstellt

| Recherchenort | Abschlußdatum der Recherche | Prüfer |
|---|---|---|
| DEN HAAG | 23-05-1989 | BOUTRUCHE J.P.E. |

EPO FORM 1503 03.82 (P0403)